(19) Europäisches Patentamt
European Patent Office
Office européen des brevets

(11) EP 1 049 456 B1

(12) **FASCICULE DE BREVET EUROPEEN**

(45) Date de publication et mention de la délivrance du brevet:
**14.07.2004 Bulletin 2004/29**

(21) Numéro de dépôt: **99900933.5**

(22) Date de dépôt: **15.01.1999**

(51) Int Cl.[7]: **A61K 7/50**, A61K 7/48

(86) Numéro de dépôt international:
**PCT/FR1999/000073**

(87) Numéro de publication internationale:
**WO 1999/036054 (22.07.1999 Gazette 1999/29)**

(54) **UTILISATION, DANS LES COMPOSITIONS COSMETIQUES, D'AGENTS TENSIOACTIFS AMPHOTERES POUR PRECIPITER, A LA DILUTION, DES POLYMERES CATIONIQUES**

VERWENDUNG AMPHOTERER TENSIDE ALS FÄLLUNGSMITTEL FÜR KATIONISCHE POLYMERE WÄHREND DES VERDÜNNENS IN KOSMETISCHEN ZUSAMMENSETZUNGEN

USE IN COSMETIC COMPOSITIONS OF AMPHOTERIC SURFACE-ACTIVE AGENTS FOR PRECIPITATING CATIONICS POLYMERS IN THE DILUED STATE

(84) Etats contractants désignés:
**AT BE CH CY DE DK ES FI FR GB GR IE IT LI LU MC NL PT SE**

(30) Priorité: **19.01.1998 FR 9800634**

(43) Date de publication de la demande:
**08.11.2000 Bulletin 2000/45**

(73) Titulaire: **RHODIA CHIMIE**
**92408 Courbevoie Cédex (FR)**

(72) Inventeur: **RICCA, Jean-Marc**
**F-75012 Paris (FR)**

(74) Mandataire: **Boittiaux, Vincent**
**Rhodia Services,**
**DPI,**
**40, rue de la Haie-Coq**
**93306 Aubervilliers Cedex (FR)**

(56) Documents cités:
**WO-A-96/37591** **WO-A-97/04743**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet l'utilisation, dans les compositions cosmétiques aqueuses transparentes destinées à être rincées, d'au moins un agent tensioactif amphotère comme agent de précipitation, à la dilution lors de l'application desdites compositions sur le cheveu et/ou la peau, de polymères cationiques hydrosolubles ou hydrodispersables dérivés de polysaccharides (notamment les celluloses cationiques et les guars cationiques hydroxyalkylés) inclus dans lesdites compositions; elle a également pour objet les compositions cosmétiques aqueuses pour le cheveu et/ou la peau, comprenant outre ledit agent tensioactif amphotère, au moins un agent tensioactif anionique, au moins un agent tensioactif choisi parmi les bétaïnes et au moins un polymère cationique hydrosoluble dérivé d'un polysaccharide.

Le cheveu ou le corps humain se salissent au contact de la pollution atmosphérique et dans une plus large mesure par des sécrétions naturelles comme le sébum. Ce phénomène nécessite un nettoyage fréquent de la peau et des cheveux à l'aide de compositions cosmétiques. Si les propriétés détergentes de celles-ci se révèlent souvent largement satisfaisantes, des lavages fréquents et répétés ont toutefois pour effet de laisser le cheveu dans un état qui nécessite un traitement complémentaire. Différentes solutions ont été proposées pour remédier à ce problème, allant de l'usage d'agents conditionneurs inclus dans la formulation cosmétique jusqu'au développement de produits spécifiques utilisés en post traitement.

Pour des raisons de coût et d'efficacité, on cherche à maximiser le dépôt des agents conditionneurs sur le cheveu et/ou la peau. Une approche générale consiste à utiliser les interactions associatives entre un agent tensioactif anionique et un polymère cationique, interactions conduisant à la précipitation d'un complexe agent tensioactif / polymère sur la surface requise. A titre d'exemple, on peut citer les formulations à base du système lauryl éther sulfate de sodium / cocamidopropyl bétaïne et chlorure d'hydroxypropyl trimonium guar comme le Jaguar C13S commercialisé par la société Rhône-Poulenc. Ce système précipite à la dilution par séparation de phase, conduisant à un dépôt accru de polymère à la surface du cheveu et/ou la peau. Toutefois ce système ne permet pas d'obtenir des formulations de transparence requise.

Une solution pour améliorer la transparence, consiste à employer, comme guar cationique, le chlorure d'hydroxypropyl hydroxypropyl trimonium guar comme le JAGUAR C162 commercialisé par la société Rhône-Poulenc, mais le système formé par association avec les agents tensioactifs précédemment cités ne précipite pas par dilution.

La Demanderesse a trouvé de façon inattendue que l'addition d'une faible quantité d'un agent tensioactif de type amphotère, à un milieu aqueux transparent contenant un agent tensioactif anionique, un agent tensioactif du type des bétaines et un polymère cationique hydrosoluble ou hydrodispersable dérivé d'un polysaccharide ne précipitant pas à la dilution par séparation de phase (celluloses cationiques et guars cationiques hydroxyalkylés notamment), permet d'obtenir, à la dilution, la précipitation dudit polymère cationique par un phénomène de séparation de phase.

En application cosmétique, cette précipitation entraîne une forte augmentation du dépôt de polymère cationique dérivé de polysaccharide sur le cheveu et/ou la peau, lorsque ledit milieu aqueux est dilué par mise en contact avec le cheveu et/ou la peau humidifiée ou mouillée.

Ce système peut aussi être utilisé pour augmenter en même temps le dépôt d'agents cosmétiques organiques non hydrosolubles pouvant être présents sous forme de dispersions de particules.

La présente invention a pour premier objet l'utilisation, dans les compositions cosmétiques aqueuses transparentes pour le cheveu et/ou la peau destinées à être rincées, d'au moins un agent tensioactif amphotère (A), en quantité de l'ordre de 0,5 à 10% en poids, de préférence de l'ordre de 0,5 à 3% en poids par rapport au poids desdites compositions cosmétiques, comme agent de précipitation, à la dilution lors de l'application desdites compositions sur le cheveu et/ou la peau, de polymère cationique hydrosoluble ou hydrodispersable dérivé d'un polysaccharide (PolC), lesdites compositions cosmétiques aqueuses transparentes comprenant au moins 90% de leur poids d'une phase aqueuse ($\Phi$) comprenant de 8 à 20% de son poids d'un système (S) formé d'au moins un agent tensioactif anionique (TAn), d'au moins un agent tensioactif choisi parmi les bétaines (TB) et d'au moins un polymère cationique hydrosoluble ou hydrodispersable dérivé d'un polysaccharide (PolC) ,

les quantités respectives des constituants (TAn), (TB) et (PolC) et la nature du polymère cationique (PolC) dans ledit système (S) étant telles que la phase aqueuse ($\phi$) constituée dudit système (S) en solution aqueuse à une concentration de 8 à 20% présente une transmittance mesurée à 600 nanomètres au moins égale à 90% et ne présente pas de comportement de séparation de phase avec formation d'un précipité à la dilution.

Selon l'invention, la phase aqueuse ($\phi$) constituée dudit système (S) en solution aqueuse à une concentration de 8 à 20% ne présente pas de comportement de séparation de phase avec formation d'un précipité à la dilution, cette même phase aqueuse ($\phi$) présentant, à la dilution, en présence de l'agent tensioactif amphotère (A), un comportement de séparation de phase avec formation d'un précipité.

Ce phénomène de séparation de phase par dilution s'opère ou se manifeste lors de l'emploi de ladite composition cosmétique par contact sur la surface à traiter (cheveu et/ou peau), surface qui présente déjà un degré d'hydratation relativement important (par exemple cheveu mouillé, peau humidifiée).

Dans le cas d'un shampooing par exemple, le facteur de dilution de la composition cosmétique lors de son application, peut être évalué à une valeur de l'ordre de 3 à 10, en considérant qu'un gramme de cheveu mouillé retient en moyenne de 0,6 à 1 g d'eau, et que d'une manière standard, 0,1 g de shampooing est appliqué par gramme de cheveu exprimé en sec, avec une durée d'application généralement de 30 à 45 secondes.
Après avoir été appliquée, la composition cosmétique doit alors être rincée pour éliminer les agents tensioactifs en excès.
Pour une bonne réalisation de l'invention ladite phase aqueuse (Φ) comprend :

- de l'ordre de 5 à 15% de son poids, de préférence de l'ordre de 8 à 12% de son poids, d'au moins un agent tensioactif anionique (TAn)
- de l'ordre de 0,5 à 10% de son poids, de préférence de l'ordre de 0,5 à 3% de son poids, d'au moins un agent tensioactif choisi parmi les bétaines (TB)
- et de l'ordre de 0,015 à 2% de son poids, de préférence de l'ordre de 0,05 à 0,5% de son poids d'au moins un polymère cationique (PolC).

Parmi les polymères cationiques (PolC) ne précipitant pas à la dilution de la phase aqueuse (ϕ) en l'absence de tensioactif amphotère (A), mais présentant en présence du tensioactif amphotère (A), un comportement de séparation de phase avec formation d'un précipité, on peut citer notamment les dérivés cationiques de la cellulose et les dérivés hydroxyalkylés des guars cationiques.
Parmi les polymères cationiques préférentiels, on peut mentionner les dérivés hydroxyalkylés (en $C_2$-$C_{22}$) des guars cationiques comme notamment le chlorure d'hydroxypropyltrimonium hydroxypropyl guar (JAGUAR C162 et JAGUAR C2000 commercialisés par Rhône-Poulenc) et les dérivés cationiques de la cellulose, comme notamment l'éther de poly(oxyéthanediyl-1,2) hydroxy-2 chlorure de triméthylammonium-3 propyl cellulose ou polyquatemium-10 (Polymer JR400 commercialisé par Union Carbide).
La cationicité de ces polymères est variable; ainsi dans le cas des dérivés hydroxypropylés de guar cationique comme les JAGUARS C162 et C2000 commercialisés par la société Rhône-Poulenc, le degré d'hydroxypropylation ("molar substitution" ou MS) sera compris entre 0,02 et 1,2 et le degré de cationicité ("degree of substitution" ou DS) sera compris entre 0,01 et 0,6. Ces produits peuvent être éventuellement fonctionnalisés par des groupes hydrophobes comme des chaînes alkyles.
Ces polymères cationiques peuvent éventuellement être fonctionnalisés par des groupements anioniques comme des groupes carboxyméthyl, sulfate, sulfonate ou phosphate, à la condition que le degré de substitution de ces groupes anioniques soit dans tous les cas inférieur au degré de substitution des groupes cationiques.
Ces polymères cationiques présentent généralement une masse moléculaire d'au moins 2000, le plus généralement de l'ordre de 200 000 à 3 000 000.
Parmi les agents tensioactifs anioniques (TAn) susceptibles d'être présents dans le cadre de cette invention, on peut citer :

- les alkylesters sulfonates de formule R-CH($SO_3$M)-COOR', où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tétraméthylammonium, diméthylpiperidinium ...) ou dérivé d'une alcanolamine (monoéthanolamine, diéthanotamine, triéthanolamine ...). On peut citer tout particulièrement les méthyl ester sulfonates dont les radical R est en $C_{14}$-$C_{16}$;
- les alkylsulfates de formule $ROSO_3$M, où R représente un radical alkyle ou hydroxyalkyle en $C_{10}$-$C_{24}$, de préférence en $C_{12}$-$C_{20}$ et tout particulièrement en $C_{12}$-$C_{18}$, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
- les alkylamides sulfates de formule RCONHR'$OSO_3$M où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
- les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB-A-1082179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates

le cation étant un métal alcalin (sodium, potassium, lithium), un reste ammonium substitué ou non substitué (méthyl-, diméthyl-, triméthyl-, tétraméthylammonium, diméthylpiperidinium ...) ou dérivé d'une alcanolamine (monoéthanolamine, diéthanolamine, triéthanolamine ... ) ;
On préfère utiliser les dérivés éthoxylés d'acides gras en particulier les dérivés de l'alcool laurique comme le lauryl éther sulfate de sodium, de magnésium, ou leurs mélanges.

Par agent tensioactif choisi parmi les bétaines (TB), on entend tout agent tensioactif portant sur la même molécule une charge négative et une charge positive permanente quel que soit le pH et ne présentant pas de point isoélectrique. Il s'agit de dérivés quaternisés.

A titre d'exemples, on peut citer :

- les bétaïnes de formule

$$R^1\ R^2\ R^3\ \overset{+}{N}R^4\ C(O)O^-$$

  comme la lauryl bétaïne (Mirataine BB de la société Rhône-Poulenc)
- les sulfo-bétaïnes de formule

$$R^1\ R^2\ R^3\overset{+}{N}R^4\ SO_3^-$$

- les amidoalkylbétaïnes de formule

$$R^1C(O)\text{ - }NH\ R^2\overset{+}{N}\ (R^3\ R^4)\ R^5\ C(O)O^-$$

  comme la cocamidopropyl bétaïne ((Mirataine BDJ de la société Rhône-Poulenc)
- les sulfo-bétaïnes de formule

$$R^1C(O)\text{ - }NH\ R^2\overset{+}{N}\ (R^3\ R^4)\ R^5\ SO_3^-$$

comme la cocamidopropyl hydroxy sultaine (Mirataine CBS de la société Rhône-Poulenc) formules dans lesquelles les radicaux $R^1$ représente un radical alkyle ou alcényle de 10 à 24 atomes de carbone, $R^2$, $R^3$, $R^4$, et $R^5$ identiques ou différents représentent un radical alkyle ou alkylène ayant de 1 à 4 atomes de carbone.

Par agent tensioactif amphotère (A), on entend tout agent tensioactif portant simultanément une charge anionique et une charge cationique, produit dont le degré d'ionisation varie en fonction du pH du milieu dans lequel il se trouve. Ces produits présentent un point isoélectrique (IEP) compris entre 3,5 et 6,5.
A titre d'exemples, on peut citer

* les alkyl ou alcényl-amphoacétates ou -amphodiacétates répondant plus généralement à la formule

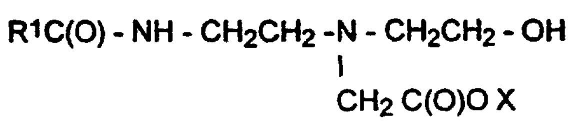

  dans laquelle $R^1$ représente un radical alkyle ou alcényle de 10 à 24 atomes de carbone, le plus souvent des chaînes coco et lauryle. (Miranol C2M et Miranol Ultra C32 de la société Rhône-Poulenc) et X un atome d'hydrogène, un métal alcalin ou alcalino-terreux, une amine
* les alkylampho-propionates ou -dipropionates, (Miranol C2M SF de la société Rhône-Poulenc)
* les alkyl amphohydroxypropyl sultaines (Miranol CS de la société Rhône-Poulenc) dont les groupes alkyles contiennent de 8 à 24 atomes de carbone, et sont le plus souvent des groupes coco ou lauryle.

Selon une variante de réalisation de l'invention. ledit agent tensioactif amphotère (A) peut également être utilisé pour augmenter le dépôt de particules de composés organiques non hydrosolubles (POins) cosmétiquement intéressants, lors de la dilution à l'emploi desdites compositions cosmétiques contenant en outre au moins une dispersion aqueuse desdites particules organiques non hydrosolubles.
Lesdites particules peuvent être présentes dans lesdites compositions à raison de l'ordre de 0,1 à 10% en poids, de

préférence de l'ordre de 0,2 à 2% en poids. Leur taille peut être comprise entre 0,15 et 70 microns.

Parmi les composés organiques insolubles cosmétiquement intéressants (POins) pouvant être présents sous forme de particules en dispersion aqueuse dans lesdites compositions cosmétiques, on peut mentionner les organopolysiloxanes non hydrosolubles et non volatils (appelés également par la suite "silicones non hydrosolubles et non volatils"), parmi lesquels on peut citer les huiles, gommes ou résines polyalkylsiloxanes, polyarylsiloxanes, polyalkylarylsiloxanes, ou leurs dérivés fonctionnalisés non hydrosolubles, ou leurs mélanges, non volatils.

Lesdits organopolysiloxanes sont considérés comme non hydrosolubles et non volatils, lorsque leur solubilité dans l'eau est inférieure à 50g/litre et leur viscosité intrinsèque d'au moins 3000 mPa.s. à 25°C.

A titre d'exemples plus particulier d'organopolysiloxanes ou silicones non hydrosolubles et non volatils, on peut citer des gommes silicones comme par exemple la gomme diphényl diméthicone commercialisée par la société Rhône-Poulenc, et de préférence les polydiméthylsiloxanes présentant une viscosité au moins égale à 600 000 mPa.s. à 25°C, et de façon encore plus préférentielle, ceux d'une viscosité supérieure à 2 000 000 mPa.s. à 25°C, tels que la Mirasil DM 500000® commercialisée par la société Rhône-Poulenc.

L'organopolysifoxane ou silicone non hydrosoluble et non volatil se trouve sous forme dispersé au sein de la composition cosmétique le renfermant. Celui-ci se présente sous forme de particules dont la taille peut être choisie en fonction de la nature de la composition cosmétique ou de la performance recherchée pour ladite composition. D'une manière générale, cette taille peut varier de 0,02 à 70 microns. D'une manière préférentielle, cette taille est de l'ordre de 1 à 80 microns, tout particulièrement de l'ordre de 1 à 30 microns.

Ces particules de silicones peuvent être constituées de mélange de silicones dont la présence peut être due au mode de mise en oeuvre de l'agent silicone cosmétiquement intéressant au sein de la composition cosmétique. Ainsi les organopolysiloxanes cosmétiquement intéressants peuvent être préalablement dispersés ou solubilisés dans des dérivés silicones de faible viscosité, volatils ou non, puis émulsionnés dans la composition cosmétique. Parmi ces silicones de faible viscosité, on peut citer les silicones volatils cycliques et les polydiméthyl siloxanes de faible masse.

On peut également mentionner comme polyorganosiloxanes, des dérivés de silicones fonctionnalisés comme les dérivés aminés, comme la Mirasil ADM-E commercialisée par la société Rhône-Poulenc, (directement mis en oeuvre sous forme d'émulsions dans la composition cosmétique ou à partir de micro émulsions préformées).

On peut également mentionner comme composés organiques insolubles pouvant être présents sous forme de particules, des huiles pouvant exercer des fonctions conditionnantes, protectrices, ou émollientes, huiles généralement choisies parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin ...) ou les huiles d'origine animale (suif, huiles de poisson ...), des dérivés de ces huiles comme les huiles hydrogénées, les dérivés de la lanoline, les huiles minérales ou les huiles paraffiniques,

le perhydrosqualane, le squalène, les diols comme le 1-2-dodécanediot, l'alcool cétylique, l'alcool stéarylique, l'alcool oléique, les esters gras comme le palmitate d'isopropyl, le cocoate d'éthyl-2-hexyl, le myristyl myristate, les esters de l'acide lactique, l'acide stéarique, l'acide béhenique, l'acide isostéarique.

On peut aussi citer des particules d'agents bactéricides ou fongicides afin d'améliorer la désinfection de la peau comme par exemple le triclosan, des agents antipelliculaires comme la zinc pyrithione ou l'octopyrox, des agents insecticides comme les pyréthroides naturels ou de synthèse. Ces différentes molécules organiques peuvent le cas échéant être préalablement encapsulées dans des matrices appropriées selon des méthodes connues de l'homme de l'art. Parmi celles-ci, on peut citer à titre d'exemple, l'encapsulation de molécules organiques dans des latex de polymères.

Les particules organiques insolubles dans l'eau peuvent également être constituées d'agents pour la protection de la peau et/ou des cheveux contre les agressions du soleil et des rayons UV comme des filtres solaires qui sont des composés chimiques absorbant fortement le rayonnement W comme les composés autorisés dans la directive Européenne N° 76/768/CEE, ses annexes et les modifications ultérieures de cette directive.

Dans le cas où les composés organiques insolubles cosmétiquement intéressants présentent une solubilité micellaire trop importante (par rapport à la quantité d'agents tensioactifs présents) ou se présentent sous forme solide à température ambiante, ceux-ci peuvent être soit mis en solution dans un véhicule organique, comme des huiles minérales ou naturelles, des dérivés de silicones, des cires, soit encapsulés dans des matnces comme des polymères (acryliques par exemple) présents en dispersion par exemple sous forme de latex.

Un deuxième objet de l'invention consiste en des compositions cosmétiques aqueuses transparentes pour le cheveu et/ou la peau destinées à être rincées, comprenant au moins 90% de leur poids d'une phase aqueuse (Φ) comprenant :

- au moins un agent tensioactif amphotère (A), en quantité de l'ordre de 0,5 à 10% en poids, de préférence de l'ordre de 0,5 à 3% en poids par rapport au poids desdites compositions cosmétiques,
- et de 8 à 20% de son poids d'un système (S) formé d'au moins un agent tensioactif anionique (TAn), d'au moins un agent tensioactif choisi parmi les bétaïnes (TB) et d'au moins un polymère cationique hydrosoluble ou hydrodispersable dérivé d'un polysaccharide (PolC) ,

les quantités respectives des constituants (TAn), (TB) et (PolC) et la nature du polymère cationique (PolC) dans ledit système (S) étant telles que la phase aqueuse (ϕ) constituée dudit système (S) en solution aqueuse à une concentration de 8 à 20% présente une transmittance mesurée à 600 nanomètres au moins égale à 90% et ne présente pas de comportement de séparation de phase avec formation d'un précipité à la dilution
ladite phase aqueuse (ϕ), en présence dudit agent tensioactif amphotère (A), présentant à la dilution un comportement de séparation de phase avec formation d'un précipité.

Pour une bonne réalisation de l'invention ladite phase aqueuse (Φ) comprend :

- de l'ordre de 5 à 15% de son poids, de préférence de l'ordre de 8 à 12% de son poids, d'au moins un agent tensioactif anionique (TAn)
- de l'ordre de 0,5 à 10% de son poids, de préférence de l'ordre de 0,5 à 3% de son poids, d'au moins un agent tensioactif choisi parmi les bétaïnes (TB)
- et de l'ordre de 0,015 à 2% de son poids, de préférence de l'ordre de 0,05 à 0,5% de son poids d'au moins un polymère cationique (PolC).

Des exemples de polymères cationiques (PolC), et d'agents tensioactifs (TAn), (TB) et (A) pouvant être présents dans lesdites compositions ont déjà été décrits ci-dessus.

On entend par le terme composition ou formulation cosmétique tous les produits ou préparations cosmétiques comme celles décrites dans l'annexe 1 ("Illustrative list by category of cosmetic products") de la directive européenne n° 761768/CEE du 27 juillet 1976, dite directive cosmétique.

Les compositions cosmétiques faisant l'objet de l'invention peuvent être formulées en un grand nombre de types de produits pour la peau et/ou le cheveu, les gels (coiffants notamment), les conditionneurs, les formulations pour le coiffage ou pour faciliter le peignage des cheveux, les formules de rinçage, les lotions pour les mains et le corps, les produits régulant l'hydratation de la peau, les laits de toilette, les compositions démaquillantes, les shampooings, les gels douche, les savons liquides et bien d'autres compositions du même type.

Selon une variante de réalisation de l'invention, lesdites compositions cosmétiques peuvent en outre contenir des particules de composés organiques non hydrosolubles (POins) cosmétiquement intéressants.
Lesdites particules peuvent être présentes dans lesdites compositions à raison de l'ordre de 0,1 à 10% en poids, de préférence de l'ordre de 0,2 à 2% en poids. Leur taille peut être comprise entre 0,15 et 70 microns.

Des exemples de constituants (POins) pouvant être présents dans lesdites compositions ont déjà été mentionnés ci-dessus.

Pour une bonne réalisation de l'invention, les compositions cosmétiques faisant l'objet de l'invention peuvent comprendre en outre des résines fixatives. Ces résines fixatives sont généralement présentes à des concentrations comprises entre 0.01 et 10%. préférentiellement entre 0.5 et 5%.

Ces résines fixatives sont préférentiellement choisies parmi les copolymères acrylate de méthyle / acrylamide, copolymères pofyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylate de méthyle, copolymères polyvinylpyrrolidone /acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate / acide méthacrylique, monométhyl éthers de poly(méthylvinyl éther/acide maléique), polyvinylacétates greffés sur des troncs polyoxyéthylènes (EP-A-219 048), les copolyesters dérivés d'acide, anhydride ou d'un diester téréphtalique et/ou isophtalique et/ou sulfoisophtalique et d'un diol, tels que :

- les copolymères polyesters à base de motifs éthylène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
- les oligomères polyesters sulfonés obtenus par sulfonation d'un oligomère dérivé de l'alcool allylique éthoxylé, du diméthyltéréphtalate et du 1,2 propylène diol (US-A-4 968 451)
- les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
- les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698)
- les monoamines ou polyamines éthoxylées, les polymères d'amines éthoxylées (US-A-4 597 898, EP-A-11 984)
- les oligomères polyesters sulfonés obtenus par condensation de l'acide isophtalique, du sulfosuccinate de diméthyle et de diéthylène glycol (FR-A-2 236 926)

- les copolymères polyesters dérivés de diméthyltéréphtalate, d'acide isophtalique, de sulfoisophtalate de diméthyl et d'éthylène glycol (EP-A-540374)
- les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).

De manière préférentielle, les résines fixatives sont du type polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.
Ces résines fixatives sont préférentiellement dispersées ou solubilisées dans le véhicule choisi.
Les compositions cosmétiques faisant l'objet de l'invention peuvent également contenir des dérivés polymériques exerçant une fonction protectrice.
Ces dérivés polymériques peuvent être présents en quantités de l'ordre de 0,01-10%, de préférence environ 0,1-5%, et tout particulièrement de l'ordre de 0,2-3% en poids, agents tels que :

- les dérivés cellulosiques tels que les hydroxyéthers de cellulose, la méthylcellulose, l'éthylcellulose, l'hydroxypropyl méthylcellulose, l'hydroxybutyl méthylcellulose
- les polyvinylesters greffés sur des troncs polyalkylènes tels que les polyvinylacétates greffés sur des troncs polyoxyéthylènes (EP-A-219 048)
- les alcools polyvinyliques

Les performances des compositions cosmétiques faisant l'objet de l'invention peuvent aussi être améliorées par l'emploi d'agents plastifiants. L'agent plastifiant pourra constituer entre 0.1 à 10% de la formulation de préférence de 1 à 10%. Parmi les agents plastifiants particulièrement utiles, on peut citer les adipates, les phtalates, les isophtalates, les azélates, les stéarates, les silicones copolyols, les glycols, l'huile de ricin, ou leurs mélanges.
On peut aussi avantageusement ajouter à ces compositions des agents séquestrants des métaux, plus particulièrement ceux séquestrants du calcium comme les ions citrates.
On peut également incorporer aux compositions cosmétiques faisant l'objet de l'invention des agents humectants, on peut citer le glycérol, le sorbitol, l'urée, le collagène, la gélatine, l'aloe vera, l'acide hyaluronique.
Pour diminuer encore l'irritation ou l'agression du cuir chevelu, on peut aussi rajouter des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation et les dérivés de ces polycarbohydrates comme les celluloses modifiées (par exemple hydroxyéthylcellulose, carboxyméthylcellulose), les dérivés du guar ou de la caroube comme leurs dérivés non-ioniques (par exemple hydroxypropylguar), les dérivés anioniques (carboxyméthyiguar et carboxyméthylhydroxypropylguar).
Des agents conservateurs comme les méthyl, éthyl, propyl et butyl esters de l'acide p-hydroxybenzoïque, le benzoate de sodium, le GERMABEN (nom de marque) ou tout agent chimique évitant la prolifération bactérienne ou des moisissures et utilisé traditionnellement des les compositions cosmétiques sont généralement introduits dans ces compositions à hauteur de 0,01 à 3% en poids. La quantité de ces produits est généralement ajustée pour éviter toute prolifération de bactéries, moisissures ou levures dans les compositions cosmétiques.
Alternativement à ces agents chimiques, on peut parfois utiliser des agents modifiants l'activité de l'eau et augmentant fortement la pression osmotique comme les carbohydrates ou des sels.
A ces ingrédients on rajoute généralement pour augmenter l'agrément lors de l'utilisation de la composition par le consommateur, un ou des parfums, des agents colorants parmi lesquels on peut citer les produits décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments. Des parfums, des colorants ou des pigments peuvent être ajoutés.
La composition peut aussi contenir des polymères viscosants ou gélifiants, comme les polyacrylates réticulés - CARBOPOL commercialisés par GOODRICH -, les dérivés de la cellulose comme l'hydroxypropylcellulose, la carboxyméthylcellulose, les guars et leurs dérivés .... utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100%.
Les compositions cosmétiques faisant l'objet de l'invention peuvent également contenir des agents dispersants polymériques en quantité de l'ordre de 0,1-7% en poids, pour contrôler la dureté en calcium et magnésium, agents tels que :

- les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maléique, acide fumarique, acide itaconique, acide aconitique, acide mesaconique, acide citraconique, acide méthylènemalonique, et tout particulièrement les polyacrylates de masse moléculaire de l'ordre de 2000 à 10 000 (US-A-3 308 067), les copolymères d'acide acrylique et d'anhydride maléique de masse moléculaire de l'ordre de 5000 à 75 000 (EP-A-66 915)
- les polyéthylèneglycols de masse moléculaire de l'ordre de 1000 à 50 000.

Les exemples suivants sont donnés à titre illustratif.

**Exemple 1 :**

Les formulations suivantes sont préparées de façon traditionnelle (pH final 6). Les quantités sont exprimées en pourcentage de matière active.

| Ingrédients | Nom INCI | Formulation | | | | | |
|---|---|---|---|---|---|---|---|
| | | 1 | 2 | 3 | 4 | 5 | 6 |
| Empicol ESB/3M (% poids) | Sodium lauryl éther (2EO) sulfate | 8 | 8 | 7 | 7 | 8 | 8 |
| Tegobetaïne L7 (% poids) | Cocamidopropyl bétaïne | 2 | 2 | 2 | 2 | 0 | 0 |
| Miranol Ultra C32 (% poids) | Sodium cocoamphoacétate | 0 | 0 | 1 | 1 | 2 | 2 |
| Jaguar C162 * (% poids) | hydroxypropyl guar hydroxypropyl trimonium chloride | 0.3 | 0 | 0.3 | 0 | 0.3 | 0 |
| Jaguar C2000 ** (% poids) | hydroxypropyl guar hydroxypropyl trimonium chloride | 0 | 0.3 | 0 | 0.3 | 0 | 0.3 |
| eau | | qsp 100 | qsp 100 | qsp 100 | qsp 100 | qsp 100 | qsp 100 |
| **Transmittance (% à 600 nm)** | | 100 | 100 | 100 | 100 | 100 | 100 |

* Jaguar C162 : hydroxypropyl guar hydroxypropyl trimonium chloride
. teneur en groupe hydroxypropyl : 0,6
. degré de substitution : 0,1

** Jaguar C2000 : hydroxypropyl guar hydroxypropyl trimonium chloride
. teneur en groupe hydroxypropyl : 0,6
. degré de substitution : 0,2

Les formulations (formules) 1 à 6 du tableau précédent, contenant environ 10% en poids d'extrait sec, sont diluées par des quantités croissantes d'eau ; la transmittance des formulations diluées est mesurée au moyen d'un spectrophotomètre Jasco 7800.
Le graphe suivant montre l'évolution de la transmittance en fonction de la concentration en matière active.

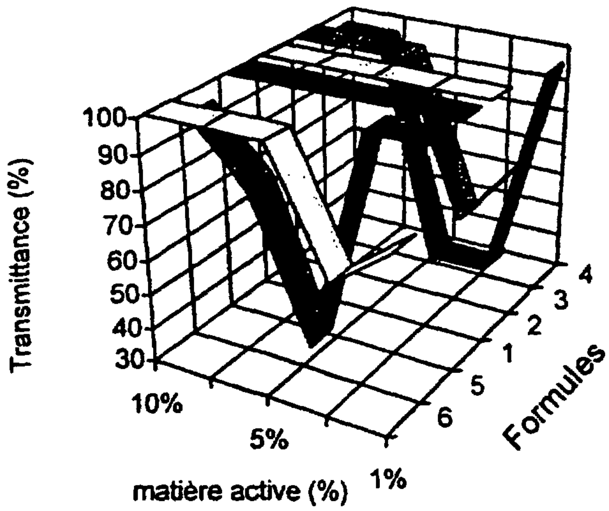


On constate que les formulations contenant du sodium cocoamphoacétate montrent la présence d'un phénomène de précipitation lors de la dilution, tandis que les formulations ne contenant que de la cocamidopropyl bétaïne restent stables à la dilution.

**Exemple 2 :**

Les formulations suivantes sont préparées de façon traditionnelle (pH final 6). Les quantités sont exprimées en pourcentage de matière active.

| Ingrédients | Nom INCI | Formulations | | | |
|---|---|---|---|---|---|
| | | **1** | **3** | **7** | **5** |
| Empicol ESB/3M (% poids) | Sodium lauryl éther (2EO) sulfate | 8 | 7 | 7 | 8 |
| Tegobetaïne L7 (% poids) | Cocamidopropyl bétaïne | 2 | 2 | 2 | 0 |
| Miranol CSE (% poids) | Sodium cocoampho hydroxypropyl sulfonate | 0 | 0 | 1 | 0 |
| Miranol Ultra C32 (% poids) | Sodium cocoamphoacetate | 0 | 1 | 0 | 2 |
| Jaguar C162 (% poids) | hydroxypropyl guar hydroxypropyl trimonium chloride | 0.3 | 0.3 | 0.3 | 0.3 |
| **Transmittance (%,600 nm)** | | 100 | 100 | 100 | 100 |

Les formulations (formules) du tableau précédent, contenant environ 10% en poids d'extrait sec, sont diluées par des quantités croissantes d'eau ; la transmittance des formulations diluées est mesurée au moyen d'un spectrophotomètre Jasco 7800.
Le graphe suivant montre l'évolution de la transmittance en fonction de la concentration en matière active.
Le graphe suivant montre l'évolution de la transmittance en fonction de la concentration.

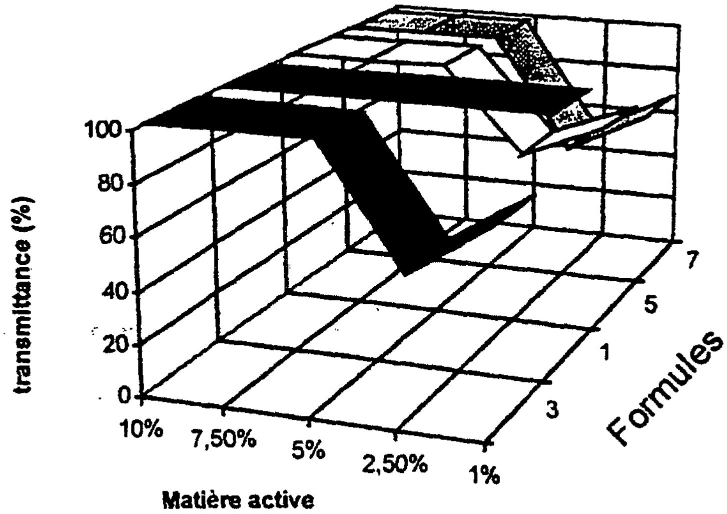


Les formulations contenant des tensioactifs amphotères montrent la présence d'un phénomène de précipitation lors de la dilution. Les formulations ne contenant que de la cocamidopropyl bétaïne restent stables à la dilution.

**Exemple 3 :**

Les formulations suivantes sont préparées de façon traditionnelle (pH final 6). Les quantités sont exprimées en pourcentage de matière active.

| **Ingrédients** | **Nom INCI** | **Formulation** | | | |
|---|---|---|---|---|---|
| | | **3** | **8** | **9** | **1** |
| Empicol ESB/3M (% poids) | Sodium lauryl éther (2EO) sulfate | 7 | 7 | 8 | 8 |
| Tegobetaïne L7 (% poids) | Cocamidopropyl bétaïne | 2 | 2 | 2 | 2 |
| Miranol Ultra C32 (% poids) | Sodium cocoamphoacétate | 1 | 1 | 0 | 0 |
| Jaguar C 162 (% poids) | hydroxypropyl guar hydroxypropyl trimonium chloride | 0.3 | 0 | 0.3 | 0 |
| Polymer JR400 (cellulose cationique) (% poids) | Polyquatemium 10 | 0 | 0.3 | 0 | 0.3 |
| **Transmittance (%, 600 nm)** | | 100 | 100 | 100 | 100 |

Les formulations (formules) du tableau précédent, contenant environ 10% en poids d'extrait sec, sont diluées par des quantités croissantes d'eau ; la transmittance des formulations diluées est mesurée au moyen d'un spectrophotomètre Jasco 7800.
Le graphe suivant montre l'évolution de la transmittance en fonction de la concentration en matière active.
Le graphe suivant montre l'évolution de la transmittance en fonction de la concentration.

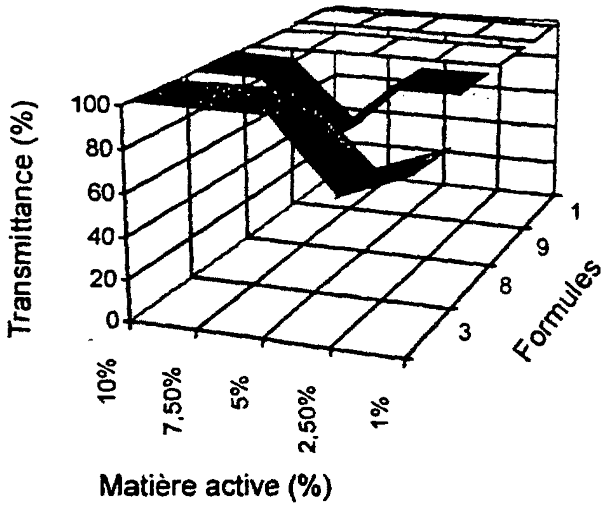


La présence de tensioactif amphotère conduit à des phénomènes de précipitation lors de la dilution.

**Exemple 4 : comparatif (guar cationique non hydroxyalkylé)**

Les formulations suivantes sont préparées de façon traditionnelle (pH final 6). Les quantités sont exprimées en pourcentage de matière active.

| **Ingrédients** | **Nom INCI** | **Formulation** | | | |
|---|---|---|---|---|---|
| | | **3** | **10** | **1** | **11** |
| Empicol ESB/3M (% poids) | Sodium lauryl éther (2EO) sulfate | 7 | 7 | 8 | 8 |
| Tegobetaïne L7 (% poids) | Cocamidopropyl bétaïne | 2 | 2 | 2 | 2 |
| Miranol Ultra C32 (% poids) | Sodium cocoamphoacétate | 1 | 1 | 0 | 0 |
| Jaguar C162 (% poids) | hydroxypropyl guar hydroxypropyl trimonium chloride | 0.3 | 0 | 0.3 | 0 |
| JAGUAR C13S (% poids) | guar hydroxypropyl trimonium chloride | 0 | 0.3 | 0 | 0.3 |
| **Transmittance (%, 600 nm)** | | 100 | <70 | 100 | <70 |
| - Jaguar C13S : guar hydroxypropyl trimonium chloride<br>. teneur en groupe hydroxypropyl : 0<br>. degré de substitution : 0,1 | | | | | |

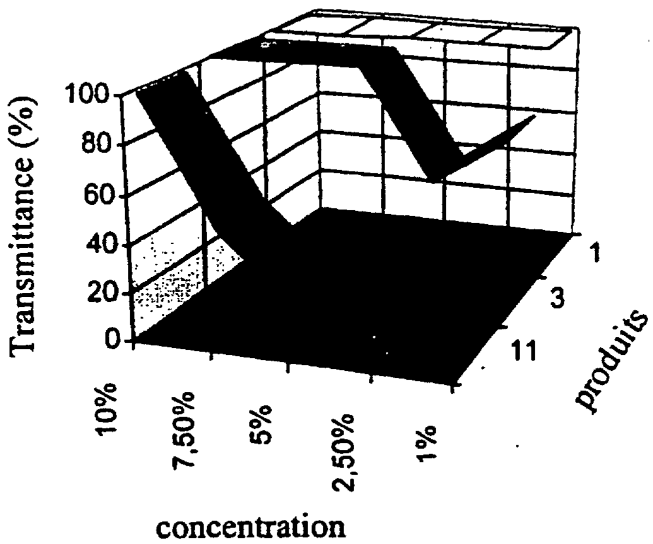


La formulation 11 contenant un guar hydroxypropyl trimonium chloride (c'est-à-dire non hydroxyalkyléné) précipite même en l'absence d'un tensioactif amphotère.

**Exemple 5 : comparatif (polymères synthétiques non polysaccharides)**

Les formulations suivantes sont préparées de façon traditionnelle (pH final 6). Les quantités sont exprimées en pourcentage de matière active.

| Ingrédients | Nom INCI | Formulation | | |
|---|---|---|---|---|
| | | 3 | 12 | 13 |
| Empicol ESB/3M (% poids) | Sodium lauryl éther (2EO) sulfate | 7 | 7 | 7 |
| Tegobetaïne L7 (% poids) | Cocamidopropyl bétaïne | 2 | 2 | 2 |
| Miranol Ultra C32 (% poids) | Sodium cocoamphoacétate | 1 | 1 | 1 |
| Jaguar C162 (% poids) | hydroxypropyl guar hydroxypropyl trimonium chloride | 0.3 | 0 | 0 |
| Mirapol 550 * (% poids) | Polyquaternium 7 | 0 | 0.3 | 0 |
| Mirapol A15 ** (% poids) | Polyquatemium 2 | 0 | 0 | 0.3 |
| **Transmittance (% à 600 nm)** | | 100 | 100 | 100 |

* Mirapol 550 : obtenu par polymérisation d'acrylamide et de chlorure de diméthyl diallylammomum

** Mirapol A15 : obtenu par condensation du bis(chloroéthyléther) de l'urée et de la N,N diméthylpropane diamine.

Les formulations (formules) du tableau précédent, contenant environ 10% en poids d'extrait sec, sont diluées par des quantités croissantes d'eau ; la transmittance des formulations diluées est mesurée au moyen d'un spectrophotomètre Jasco 7800.

Le graphe suivant montre l'évolution de la transmittance en fonction de la concentration en matière active.

On constate que les formulations contenant du Mirapol 550 et Mirapol A15, polymères cationiques ne dérivant pas de polysaccharides ne précipitent pas à la dilution.

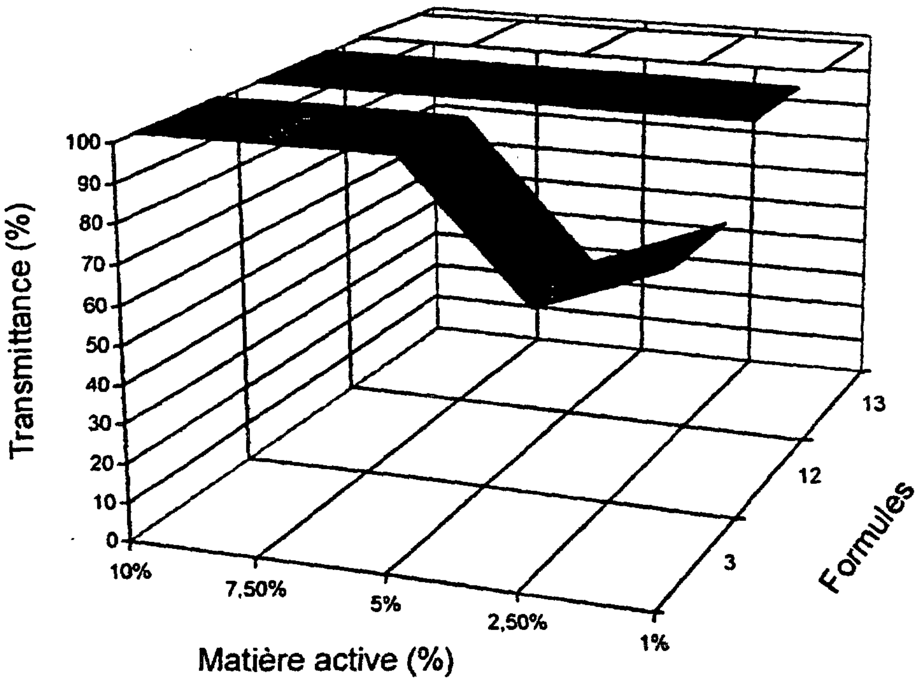


**Exemple 6 : Analyse sensorielle**

Les formulations suivantes sont préparées de façon traditionnelle (pH final 6). Les quantités sont exprimées en pourcentage de matière active.

| Ingrédients | Nom INCI | Formulation | |
|---|---|---|---|
| | | **1** | **3** |
| Empicol ESB/3M (% poids) | Sodium lauryl éther (2EO) sulfate | 8 | 7 |
| Tegobetaïne L7 (% poids) | Cocamidopropyl bétaïne | 2 | 2 |
| Miranol Ultra C32 (% poids) | Sodium cocoamphoacetate | 0 | 1 |
| Jaguar C162 (% poids) | hydroxypropyl guar hydroxypropyl trimonium chloride | 0.3 | 0.3 |
| **Transmittance à 10% actif (% à 600 nm)** | | 100 | 100 |
| **Transmittance à 2,5% actif (% à 600 nm)** | | 100 | 40 |

Ces formulations sont évaluées par analyse sensorielle (panel 25 juges).
Les mèches (cheveu européen) de 7 sont préalablement prétraitées par une solution de lauryl sulfate (5%) pendant une heure. Les mèches sont alors rincées à l'eau puis séchées.
Les mèches sont ensuite lavées avec 0,7 g de formulation pendant 30 secondes, puis rincées pendant 45 secondes. Les mèches sont alors mises sur rouleaux puis séchées.
Les résultats d'évaluation sont repris dans le graphe suivant :

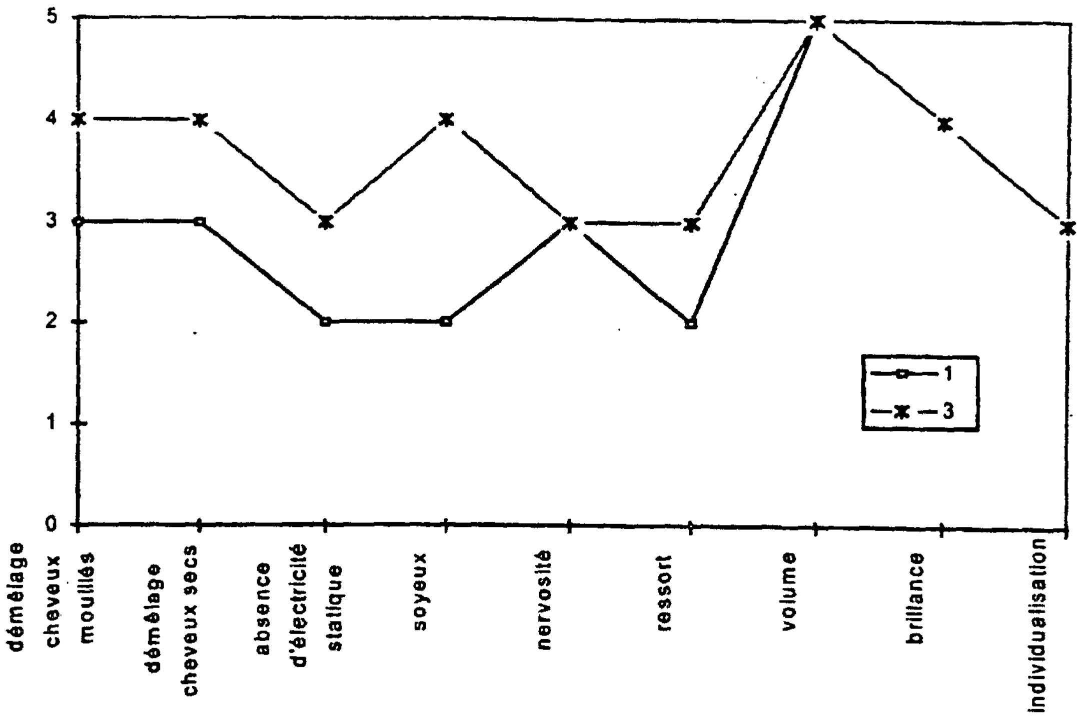


Le phénomène de précipitation contrôlée permet d'améliorer le démêlage, l'absence d'électricité statique, et le soyeux.

**Exemple 7 : augmentation de dépôt**

Les formulations suivantes sont préparées de façon traditionnelle (pH final 6). Les quantités sont exprimées en pourcentage de matière active.

| **Ingrédients** | **Nom INCI** | **Formulation** | |
|---|---|---|---|
| | | **1** | **3** |
| Empicol ESB/3M (% poids) | Sodium lauryl éther (2EO) sulfate | 8 | 7 |
| Tegobetaïne L7 (% poids) | Cocamidopropyl bétaïne | 2 | 2 |
| Miranol Ultra C32 (% poids) | Sodium cocoamphoacetate | 0 | 1 |
| Jaguar C162 (% poids) | hydroxypropyl guar hydroxypropyl trimonium chloride | 0.3 | 0.3 |
| Rheozan (% poids) | Succinoglycan gum | 0.1 1 | 0.1 1 |
| Mirasil DME-30 (% poids) | Dimethicone emulsion (+) succinoglycan gum | 2 | 2 |

Des mèches de cheveux européens (4 g) sont préalablement rincées, puis traitées par une solution de lauryl sulfate (10%) pendant 60 secondes. Les mèches sont alors rincées pendant 60 secondes. Cette opération est répétée deux fois.
Les mèches sont ensuite lavées avec 0,25 g de formulation pendant 30 secondes, puis rincées pendant 60 secondes.
Les mèches sont ensuite séchées à l'air pendant une heure
La quantité de silicone déposée est dosée après alcoolyse du polymère déposé et dosage des résidus volatils par chromatographie en phase gazeuse.
Les résultats sont exprimés en rendement de dépôt.
Le rendement de dépôt de la formulation 1 est de 4%, celle de la formulation 3 de 8%.
La présence d'une faible quantité de tensioactif amphotère permet donc de doubler la quantité de silicone déposée

sur le cheveu.

**Exemple 8: shampooing conditionneur**

Les quantités sont exprimées en pourcentage de produit tel que.

| Ingrédients | Nom INCI | % poids |
|---|---|---|
| Empicol ESB/3M | Sodium lauryl éther sulfate | 25 |
| Tegobetaïne L7 | Cocamidopropyl bétaïne | 6,7 |
| Miranol Ultra C32 | Sodium cocoamphoacetate | 3,2 |
| Jaguar C162 | Hydroxypropyl guar hydroxypropyl trimonium chloride | 0.3 |
| acide citrique | Citric acid | qs pH 6 |
| parfum | Parfum | qs |
| Conservateur | - | qs |
| eau | Aqua | qsp 100 |

PROCEDURE

Disperser le Jaguar C162 dans l'eau et hydrater avec de l'acide citrique. Ajouter le Miranol Ultra C32, la Tegobetaïne L7, puis l'Empicol ES83/M sous agitation. Ajuster le pH avec l'acide citrique à 6,5.

**Exemple 9 : gel douche transparent**

Les quantités sont exprimées en pourcentage de produit tel que.

| Ingrédients | nom INCI | % poids |
|---|---|---|
| Jaguar C162 | hydroxypropyl guar hydroxypropyl trimonium chloride | 0,2 |
| Mirasil ADM-E | Amodimethicone (and) trideceth-6 | 2 |
| Miranol Ultra C32 | Sodium cocoamphoacetate | 14 |
| Empicol ESB3/M | Sodium laureth sulfate | 34 |
| Mirataine CBS | Cocamidopropyl hydroxy sultaine | 3 |
| conservateur | - | qs |
| parfum | parfum | 0,2 |
| Tween 20 | Polysorbate 20 | 0,2 |
| acide citrique | citric acid | 2,3 |
| chlorure de sodium | sodium chloride | qs |
| eau | aqua | qs 100 |

PROCEDURE

Disperser le Jaguar C162 dans l'eau et hydrater avec de l'acide citrique. Ajouter les autres ingrédients sous agitation et dans l'ordre indiqué. Ajuster le pH avec l'acide citrique à 6,5.

**Exemple 10 : gel douche**

Les quantités sont exprimées en pourcentage de produit tel que.

| Ingrédients | Nom INCI | % poids |
|---|---|---|
| Empicol ESB/3M | Sodium lauryl éther sulfate | 25 |
| Tegobetaïne L7 | Cocamidopropyl bétaïne | 6,7 |
| Miranol CSE | Sodium cocoampho hydroxypropyl sulfonate | 3,2 |
| Polymer JR400 | Polyquaternium-10 | 0.3 |
| acide citrique | Citric acid | qs pH 6 |
| parfum | Parfum | qs |
| Conservateur | - | qs |
| eau | Aqua | qs 100 |

PROCEDURE

Disperser le Polymer JR400 dans l'eau et ajouter le Miranol CSE, la Tegobetaïne L7, puis l'Empicol ESB3/M sous agitation. Ajuster le pH avec l'acide citrique à 6,5.

## Revendications

1. Utilisation, dans les compositions cosmétiques aqueuses transparentes pour le cheveu et/ou la peau destinées à être rincées, d'au moins un agent tensioactif amphotère (A), en quantité de 0,5 à 10% en poids, de préférence de 0,5 à 3% en poids par rapport au poids desdites compositions cosmétiques, comme agent de précipitation, à la dilution lors de l'application desdites compositions sur le cheveu et/ou la peau, de polymère cationique hydrosoluble ou hydrodispersable dérivé d'un polysaccharide (PolC),
lesdites compositions cosmétiques aqueuses transparentes comprenant au moins 90% de leur poids d'une phase aqueuse (Φ) comprenant de 8 à 20% de son poids d'un système (S) formé d'au moins un agent tensioactif anionique (TAn), d'au moins un agent tensioactif choisi parmi les bétaïnes (TB) et d'au moins un polymère cationique hydrosoluble ou hydrodispersable dérivé d'un polysaccharide (PolC) ,
les quantités respectives des constituants (TAn), (TB) et (PolC) et la nature du polymère cationique (PolC) dans ledit système (S) étant telles que la phase aqueuse (ϕ) constituée dudit système (S) en solution aqueuse à une concentration de 8 à 20% présente une transmittance mesurée à 600 nanomètres au moins égale à 90% et ne présente pas de comportement de séparation de phase avec formation d'un précipité à la dilution,
ledit agent tensioactif amphotère (A) étant choisi parmi les alkyl ou alcényl-amphoacétates ou -amphodiacétates, les alkylampho-propionates ou -dipropionates, les alkyl amphohydroxypropyl sultaines dont les groupes alkyles contiennent de 8 à 24 atomes de carbone.

2. Utilisation selon la revendication 1) **caractérisée en ce que** ladite phase aqueuse (Φ) comprend :

    - de 5 à 15% de son poids, de préférence de l'ordre de 8 à 12% de son poids, d'au moins un agent tensioactif anionique (TAn)
    - de 0,5 à 10% de son poids, de préférence de l'ordre de 0,5 à 3% de son poids, d'au moins un agent tensioactif choisi parmi les bétaïnes (TB)
    - et de 0,015 à 2% de son poids, de préférence de l'ordre de 0,05 à 0,5% de son poids d'au moins un polymère cationique (PolC).

3. Utilisation selon la revendication 1) ou 2), **caractérisée en ce que** ledit polymère cationique (PolC) est choisi parmi les dérivés cationiques de la cellulose et les dérivés hydroxyalkylés des guars cationiques.

4. Utilisation selon la revendication 3) **caractérisée en ce que** ledit polymère cationique (PolC) est le chlorure d'hydroxypropyltrimonium hydroxypropyl guar ou l'éther de poly(oxyéthanediyl-1,2) hydroxy-2 chlorure de triméthylammonium-3 propyl cellulose.

5. Utilisation selon l'une quelconque des revendications 1) à 4), **caractérisée en ce que** ledit agent tensio-actif anionique (TAn) est choisi parmi les alkylesters sulfonates, les alkylsulfates et leurs dérivés éthoxylénés et/ou

propoxylénés, les alkylamides sulfates, et leurs dérivés éthoxylénés et/ou propoxylénés, les sels d'acides gras saturés ou insaturés.

**6.** Utilisation selon l'une quelconque des revendications 1) à 5), **caractérisée en ce que** ledit agent tensio-actif (TB) est choisi parmi les bétaïnes, les sulfo-bétaïnes, les amidoalkylbétaïnes.

**7.** Utilisation selon l'une quelconque des revendications 1) à 6), dudit agent tensioactif amphotère (A) dans lesdites compositions cosmétiques contenant en outre au moins un composé organique non hydrosoluble (POins), comme agent de précipitation dudit polymère cationique (PolC) et comme agent de dépôt de particules dudit composé organique non hydrosoluble (POins), à la dilution.

**8.** Utilisation selon la revendication 7), **caractérisée en ce que** composé organique non hydrosoluble (POins) est présent dans lesdites compositions à raison de 0,1 à 10%, de préférence de 0,2 à 2% en poids.

**9.** Utilisation selon la revendication 7) ou 8), **caractérisée en ce que** ledit composé organique non hydrosoluble (POins) est choisi parmi les organopolysiloxanes non hydrosolubles et non volatils, les huiles exerçant des fonctions conditionnantes, protectrices ou émollientes, les agents bactéricides ou fongicides, les filtres solaires.

**10.** Compositions cosmétiques aqueuses transparentes pour le cheveu et/ou la peau destinées à être rincées, comprenant

* au moins 90% de leur poids d'une phase aqueuse (Φ) comprenant

  - au moins un agent tensioactif amphotère (A), en quantité de 0,5 à 10% en poids, de préférence de 0,5 à 3% en poids par rapport au poids desdites compositions cosmétiques, choisi parmi les alkyl ou alcénylamphoacétates ou -amphodiacétates, les alkylampho-propionates ou -dipropionates, les alkyl amphohydroxypropyl sultaines dont les groupes alkyles contiennent de 8 à 24 atomes de carbone,
  - et de 8 à 20% de son poids d'un système (S) formé d'au moins un agent tensioactif anionique (TAn), d'au moins un agent tensioactif choisi parmi les bétaïnes (TB) et d'au moins un polymère cationique hydrosoluble ou hydrodispersable dérivé d'un polysaccharide (PolC) ,

  les quantités respectives des constituants (TAn), (TB) et (PolC) et la nature du polymère cationique (PolC) dans ledit système (S) étant telles que la phase aqueuse (ϕ) constituée dudit système (S) en solution aqueuse à une concentration de 8 à 20% présente une transmittance mesurée à 600 nanomètres au moins égale à 90% et ne présente pas de comportement de séparation de phase avec formation d'un précipité à la dilution, ladite phase aqueuse (ϕ), en présence dudit agent tensioactif amphotère (A), présentant à la dilution un comportement de séparation de phase avec formation d'un précipité.
* et éventuellement au moins un composé organique non hydrosoluble (POins).

**11.** Compositions cosmétiques selon la revendication 10), **caractérisées en ce que** ladite phase aqueuse (Φ) comprend :

- de 5 à 15% de son poids, de préférence de l'ordre de 8 à 12% de son poids, d'au moins un agent tensioactif anionique (TAn)
- de 0,5 à 10% de son poids, de préférence de l'ordre de 0,5 à 3% de son poids, d'au moins un agent tensioactif choisi parmi les bétaïnes (TB)
- de 0,015 à 2% de son poids, de préférence de l'ordre de 0,05 à 0,5% de son poids d'au moins un polymère cationique (PolC).

**12.** Compositions cosmétiques selon la revendication 10) ou 11), **caractérisées en ce que** lesdits polymères cationiques (PolC), agents tensioactifs (TAn), et (TB) et les éventuels composés organiques non hydrosolubles (POins) sont choisis parmi ceux mentionnés aux revendications 3), 4), 5), 6) ou 9).

**13.** Compositions selon l'une quelconque des revendications 10) à 12), **caractérisées en ce qu'**elles comprennent en outre au moins un additif choisi parmi les résines fixatives, les polymères exerçant une action protectrice, les agents plastifiants, séquestrants des métaux, humectants, des polymères hydrosolubles ou hydrodispersables, des agents conservateurs, des parfums, des colorants, des pigments, des polymères viscosifiants ou gélifiants, des dispersants polymériques.

## Patentansprüche

1. Verwendung in wäßrigen, transparenten kosmetischen Zusammensetzungen für das Haar und/oder die Haut, die zum Spülen vorgesehen sind, von mindestens einem amphoteren oberflächenaktiven Mittel (A) in einer Menge von 0,5 bis 10 Gew.-%, vorzugsweise 0,5 bis 3 Gew.-%, bezogen auf das Gewicht der genannten kosmetischen Zusammensetzungen, als Fällungsmittel von wasserlöslichem oder wasserdispergierbarem kationischem Polymer, abgeleitet von einem Polysaccharid (PolC), bei der Verdünnung und im Fall der Anwendung der genannten Zusammensetzungen auf dem Haar und/oder der Haut, wobei die genannten wäßrigen, transparenten kosmetischen Zusammensetzungen mindestens zu 90 % ihres Gewichtes eine wäßrige Phase (Φ) umfassen, die ihrerseits 8 bis 20 % ihres Gewichtes ein System (S) umfaßt, gebildet von mindestens einem anionischen oberflächenaktiven Mittel (TAn), mindestens einem oberflächenaktiven Mittel, ausgewählt unter den Betaïnen (TB), und mindestens einem wasserlöslichen oder wasserdispergierbaren kationischen Polymer, abgeleitet von einem Polysaccharid (PolC), und wobei die jeweiligen Mengen der Bestandteile (TAn), (TB) und (PolC) und die Beschaffenheit des kationischen Polymers (PolC) in dem genannten System (S) derart sind, daß die wäßrige Phase (Φ), gebildet von dem genannten System (S) in wäßriger Lösung bei einer Konzentration von 8 bis 20 %, einen Transmissionsgrad, gemessen bei 600 nm, von mindestens gleich 90 % aufweist und kein Verhalten der Phasentrennung mit Bildung eines Niederschlages bei der Verdünnung zeigt, und wobei das genannte amphotere oberflächenaktive Mittel (A) ausgewählt wird unter den Alkyl- oder Alkenyl-amphoacetaten oder -amphodiacetaten, den Alkyl-amphopropionaten oder -dipropionaten, den Alkyl-amphohydroxypropyl-sultainen, deren Alkylgruppen 8 bis 24 Kohlenstoffatome enthalten.

2. Verwendung nach Anspruch 1, **dadurch gekennzeichnet, daß** die genannte wäßrige Phase (Φ) umfaßt:

   - 5 bis 15 % ihres Gewichtes, vorzugsweise in der Größenordnung von 8 bis 12 % ihres Gewichtes, von mindestens einem anionischen oberflächenaktiven Mittel (TAn),
   - 0,5 bis 10 % ihres Gewichtes, vorzugsweise in der Größenordnung von 0,5 bis 3 % ihres Gewichtes, von mindestens einem oberflächenaktiven Mittel (TB), ausgewählt unter den Betaïnen,
   - und 0,015 bis 2 % ihres Gewichtes, vorzugsweise in der Größenordnung von 0,05 bis 0,5 % ihres Gewichtes, von mindestens einem kationischen Polymer (PolC).

3. Verwendung nach Anspruch 1 oder 2, **dadurch gekennzeichnet, daß** das genannte kationische Polymer (PolC) ausgewählt wird unter den kationischen Derivaten von Cellulose und den hydroxyalkylierten Derivaten von kationischen Guars.

4. Verwendung nach Anspruch 3, **dadurch gekennzeichnet, daß** das genannte kationische Polymer (PolC) das Chlorid von Hydroxypropyltrimonium-hydroxypropyl-guar oder der Ether von Poly-(1,2-oxyethandiyl)-2-hydroxychlorid von 3-Trimethylammonium-propylcellulose ist.

5. Verwendung nach irgendeinem der Ansprüche 1 bis 4, **dadurch gekennzeichnet, daß** das genannte anionische oberflächenaktive Mittel (TAn) ausgewählt wird unter den Alkylester-sulfonaten, den Alkylsulfaten und ihren ethoxylenischen und/oder propoxylenischen Derivaten, den Alkylamid-sulfaten und ihren ethoxylenischen und/oder propoxylenischen Derivaten, den Salzen von gesättigten oder ungesättigten Fettsäuren.

6. Verwendung nach irgendeinem der Ansprüche 1 bis 5, **dadurch gekennzeichnet, daß** das genannte oberflächenaktive Mittel (TB) ausgewählt wird unter den Betaïnen, den Sulfobetaïnen, den Amidoalkylbetaïnen.

7. Verwendung nach irgendeinem der Ansprüche 1 bis 6, wobei das genannte amphotere oberflächenaktive Mittel (A) in den genannten kosmetischen Zusammensetzungen außerdem mindestens eine nicht wasserlösliche organische Verbindung (POins) enthält, als Fällungsmittel des genannten kationischen Polymers (PolC) und als Ablagerungsmittel der Teilchen der genannten nicht wasserlöslichen organischen Verbindung (POins) bei der Verdünnung.

8. Verwendung nach Anspruch 7, **dadurch gekennzeichnet, daß** die nicht wasserlösliche organische Verbindung (POins) in den genannten Zusammensetzungen im Verhältnis von 0,1 bis 10 Gew.-%, vorzugsweise 0,2 bis 2 Gew.-% anwesend ist.

9. Verwendung nach Anspruch 7 oder 8, **dadurch gekennzeichnet, daß** die genannte nicht wasserlösliche organische Verbindung (POins) ausgewählt wird unter den nicht wasserlöslichen und nicht flüchtigen Organopolysiloxanen, den Ölen mit konditionierenden, schützenden oder weichmachenden Funktionen, den bakteriziden oder fungiziden Mitteln, den Solarfiltern.

**10.** Wäßrige, transparente kosmetische Zusammensetzungen für das Haar und/oder die Haut, die zum Spülen vorgesehen sind, umfassend

* mindestens zu 90 % ihres Gewichtes eine wäßrige Phase (Φ), umfassend

  - mindestens ein amphoteres oberflächenaktives Mittel (A) in einer Menge von 0,5 bis 10 Gew.-%, vorzugsweise 0,5 bis 3 Gew.-%, bezogen auf das Gewicht der genannten kosmetischen Zusammensetzungen, ausgewählt unter den Alkyl- oder Alkenyl-amphoacetaten oder -amphodiacetaten, den Alkyl-amphopropionaten oder -dipropionaten, den Alkyl-amphohydroxypropyl-sultainen, deren Alkylgruppen 8 bis 24 Kohlenstoffatome enthalten, und
  - 8 bis 20 % ihres Gewichtes ein System (S), gebildet von mindestens einem anionischen oberflächenaktiven Mittel (TAn), mindestens einem oberflächenaktiven Mittel, ausgewählt unter den Betaïnen (TB), und mindestens einem wasserlöslichen oder wasserdispergierbaren kationischen Polymer, abgeleitet von einem Polysaccharid (PolC),

wobei die jeweiligen Mengen der Bestandteile (TAn), (TB) und (PolC) und die Beschaffenheit des kationischen Polymers (PolC) in dem genannten System (S) derart sind, daß die wäßrige Phase (Φ), gebildet von dem genannten System (S) in wäßriger Lösung bei einer Konzentration von 8 bis 20 % einen Transmissionsgrad, gemessen bei 600 nm, von mindestens gleich 90 % aufweist und kein Verhalten der Phasentrennung mit Bildung eines Niederschlages bei der Verdünnung zeigt, und wobei die genannte wäßrige Phase (Φ) in Anwesenheit des genannten amphoteren oberflächenaktiven Mittels (A) bei der Verdünnung ein Verhalten der Phasentrennung mit Bildung eines Niederschlages aufweist,

- und gegebenenfalls mindestens eine nicht wasserlösliche organische Verbindung (POins).

**11.** Kosmetische Zusammensetzungen nach Anspruch 10, **dadurch gekennzeichnet, daß** die genannte wäßrige Phase (Φ) umfaßt:

- 5 bis 15 % ihres Gewichtes, vorzugsweise in der Größenordnung von 8 bis 12 % ihres Gewichtes, von mindestens einem anionischen oberflächenaktiven Mittel (TAn),
- 0,5 bis 10 % ihres Gewichtes, vorzugsweise in der Größenordnung von 0,5 bis 3 % ihres Gewichtes, von mindestens einem oberflächenaktiven Mittel (TB), ausgewählt unter den Betaïnen,
- 0,015 bis 2 % ihres Gewichtes, vorzugsweise in der Größenordnung von 0,05 bis 0,5 % ihres Gewichtes, von mindestens einem kationischen Polymer (PolC).

**12.** Kosmetische Zusammensetzungen nach Anspruch 10 oder 11, **dadurch gekennzeichnet, daß** die genannten kationischen Polymere (PolC), die oberflächenaktiven Mittel (TAn) und (TB) und die eventuellen nicht wasserlöslichen organischen Verbindungen (POins) unter denen ausgewählt werden, die in den Ansprüchen 3, 4, 5, 6 oder 9 erwähnt sind.

**13.** Zusammensetzungen nach irgendeinem der Ansprüche 10 bis 12, **dadurch gekennzeichnet, daß** sie außerdem mindestens einen Zusatzstoff umfassen, ausgewählt unter den Fixativ-Harzen, den Polymeren mit einer Schutzwirkung, den Weichmachern, den Maskierungsmitteln für Metalle, den Netzmitteln, den wasserlöslichen oder wasserdispergierbaren Polymeren, den Konservierungsstoffen, den Parfums, den Farbstoffen, den Pigmenten, den viskositätssteigernden oder gelbildenden Polymeren, den polymerischen Dispergierungsmitteln.

## Claims

**1.** Use, in transparent aqueous cosmetic compositions for the hair and/or skin intended to be rinsed, of at least one amphoteric surfactant (A) in a quantity of from 0.5% to 10% by weight, preferably of 0.5% to 3% by weight with respect to the weight of said cosmetic compositions, as an agent for precipitating a hydrosoluble or hydrodispersible cationic polymer derivative of a polysaccharide (PolC) on dilution during application of said compositions to the hair and/or skin; at least 90% of the weight of said transparent aqueous cosmetic compositions being comprised by an aqueous phase (Φ) with 8% to 20% of its weight being comprised by a system (S) formed by at least one

anionic surfactant (TAn), at least one surfactant selected from betaines (TB) and at least one hydrosoluble or hydrodispersible cationic polymer derived from a polysaccharide (PolC); the respective quantities of constituents (TAn), (TB) and (PolC) and the nature of the cationic polymer (PolC) in said system (S) being such that the transmittance of the aqueous phase (Φ) constituted by said system (S) in aqueous solution in a concentration of 8% to 20%, measured at 600 nanometres, is at least 90% and does not phase separate with formation of a precipitate on dilution,
said amphoteric surfactant (A) being selected from alkyl or alkenyl-amphoacetates or amphodiacetates, alkylampho-propionates or -dipropionates, and alkylamphohydroxypropyl sultaines, wherein the alkyl groups contain 8 to 24 carbon atoms.

**2.** Use according to claim 1, **characterized in that** said aqueous phase (Φ) comprises:

- from 5% to 15% of its weight, preferably of the order of 8% to 12% of its weight, of at least one anionic surfactant (TAn);
- from 0.5% to 10% of its weight, preferably of the order of 0.5% to 3% of its weight, of at least one surfactant selected from betaines (TB);
- and from 0.015% to 2% of its weight, preferably of the order of 0.05% to 0.5% of its weight, of at least one cationic polymer (PolC).

**3.** Use according to claim 1 or claim 2, **characterized in that** said cationic polymer (PolC) is selected from cationic cellulose derivatives and hydroxyalkylated cationic guar derivatives.

**4.** Use according to claim 3, **characterized in that** said cationic polymer (PolC) is hydroxypropyl guar hydroxypropyltrimonium chloride or cellulose,2-(2-hydroxy-3-(trimethylammonium)propoxy)ethyl ether, chloride.

**5.** Use according to any one of claims 1 to 4, **characterized in that** said anionic surfactant (TAn) is selected from alkylester sulphonates, alkylsulphates and their ethoxylenated and/or propoxylenated derivatives, alkylamide sulphates, and their ethoxylenated and/or propoxylenated derivatives, and salts of saturated or unsaturated fatty acids.

**6.** Use according to any one of claims 1 to 5, **characterized in that** said surfactant (TB) is selected from betaines, sulphobetaines and amidoalkylbetaines.

**7.** Use according to any one of claims 1 to 6 said amphoteric surfactant (A) in said cosmetic compositions further containing at least one non hydrosoluble organic compound (POins) as an agent for precipitating said cationic polymer (PolC) and as an agent for depositing particles of said non hydrosoluble organic compound (POins) on dilution.

**8.** Use according to claim 7, **characterized in that** the non hydrosoluble organic compound (POins) is present in said compositions in an amount of 0.1 % to 10%, preferably 0.2% to 2% by weight.

**9.** Use according to claim 7 or claim 8, **characterized in that** said non hydrosoluble organic compound (POins) is selected from non hydrosoluble and non volatile organopolysiloxanes, oils exerting conditioning, protective or emollient functions, bactericidal or fungicidal agents, and sun screens.

**10.** Transparent aqueous cosmetic compositions for the hair and/or skin intended to be rinsed, comprising:

- at least 90% of their weight of an aqueous phase (Φ) comprising:

  - at least one amphoteric surfactant (A) in a quantity of the order of 0.5% to 10% by weight, preferably of the order of 0.5% to 3% by weight, with respect to the weight of same cosmetic compositions, selected from alkyl or alkenyl-amphoacetates or ampho-diacetates, alkylampho-propionates or -dipropionates,and alkyl amphohydroxypropyl sultaines, the alkyl groups of which contain 8 to 24 carbon atoms;
  - and 8% to 20% of its weight of a system (S) formed by at least one anionic surfactant (TAn), at least one surfactant selected from betaines (TB) and at least one hydrosoluble or hydrodispersible cationic polymer derived from a polysaccharide (PolC);

  the respective quantities of constituents (TAn), (TB) and (PolC) and the nature of the cationic polymer (PolC)

in said system (S) being such that the transmittance of the aqueous phase (Φ) constituted by said system (S) in aqueous solution in a concentration of 8% to 20%, measured at 600 nanometres, is at least 90% and does not phase separate with formation of a precipitate on dilution, said aqueous phase (Φ) exhibiting phase separation behaviour with formation of a precipitate on dilution in the presence of said amphoteric surfactant (A);
- and optionally at least one non hydrosoluble organic compound (POins).

**11.** Cosmetic compositions according to claim 10, **characterized in that** said aqueous phase (Φ) comprises:

- from 5% to 15% of its weight, preferably of the order of 8% to 12% of its weight, of at least one anionic surfactant (TAn);
- from 0.5% to 10% of its weight, preferably of the order of 0.5% to 3% of its weight, of at least one surfactant selected from betaines (TB);
- and from 0.015% to 2% of its weight, preferably of the order of 0.05% to 0.5% of its weight, of at least one cationic polymer (PolC).

**12.** Cosmetic compositions according to claim 10 or claim 11, **characterized in that** said cationic polymers (PolC), surfactants (TAn), (TB) and (A) and optional non hydrosoluble organic compounds (POins) are selected from those defined in claims 3, 4, 5, 6, or 9.

**13.** Compositions according to any one of claims 10 to 12, **characterized in that** they further comprise at least one additive selected from fixative resins, polymers exerting a protective action, plasticisers, metal sequestrating agents, moistening agents, hydrosoluble or hydrodispersible polymers, preservatives, fragrances, colouring agents, pigments, viscosifying or gelling polymers, and polymeric dispersing agents.